# EUROPÄISCHE PATENTSCHRIFT

(11) **EP 3 831 994 B1**
(45) Veröffentlichungstag und Bekanntmachung des Hinweises auf die Patenterteilung: **09.04.2025**
(21) Anmeldenummer: 19020672.2
(22) Anmeldetag: 03.12.2019
(51) Int. Cl.: D04B 1/26, A61F 13/08, D04B 1/10

(54) **GESTRICKTEIL**
KNITTED PIECE
PIÈCE TRICOTÉE

(43) Veröffentlichungstag der Anmeldung: 09.06.2021
(73) Patentinhaber: medi GmbH & Co. KG, 95448 Bayreuth (DE)
(72) Erfinder: Huger, Christa, 95326 Kulmbach (DE)

(56) Entgegenhaltungen:
- CA-A- 702 307
- DE-A1- 1 760 822
- DE-A1- 1 802 491
- DE-A1- 102006 048 313
- DE-A1- 4 103 383
- US-A- 3 274 804
- US-A- 3 479 844

## Beschreibung

Die vorliegende Erfindung bezieht sich auf ein Gestrickteil, vorzugsweise auf ein kompressives Gestrickteil, nach dem Oberbegriff des Anspruch 1.

Derartige Gestrickteile werden, insbesondere für die Ausbildung von Arm- oder Beinbekleidungsstücken, beispielsweise als Arm- oder Beinstrumpf, verwendet. Für den medizinischen Einsatz und/ oder für die Anwendung bei sportlichen Aktivitäten weisen diese oftmals einen oder mehrere kompressive Bereiche auf, welche dazu dienen gezielt Druck auf den Körper eines Patienten auszuüben. Der auf den Körper eines Patienten ausgeübte Druck wird als Kompression bezeichnet. Ziel bei Kompressionsstrümpfen beispielsweise, insbesondere in Form von Beinlingen mit oder ohne Hand- oder Fußteil für den medizinischen Einsatz, ist es unter anderem ein geschädigtes Venen- und/ oder Lymphsystem eines Patienten zu entlasten. Durch den zugeführten Druck wird eine zunehmende Schwellung der Gliedmaßen vermieden, der Abtransport von venösem Blut und Lymphe verbessert und die Blutzufuhr gesteigert. Bei Einsatz von Kompressionsstrümpfen in dem Sportbereich bewirken diese eine Leistungssteigerung bzw. eine verbesserte Regeneration.

Zur Ausbildung dieser kompressiven und nicht-kompressiven Gestrickteile, werden diese vorzugsweise mittels einer Rundstrickmaschine rundgestrickt. Hierzu werden die Gestrickteile aus wenigstens einem maschenbildenden Strickfaden gestrickt. Zur Bildung kompressiver Gestrickteile wird in die gestrickten Maschen ein elastischer Schussfaden in jeder oder in jede X-te Reihe eingelegt. Zur Herstellung, insbesondere zur Messung und Gütesicherung, von kompressiven Arm- oder Beinstrümpfen für die medizinische Anwendung, existiert die RAL-GZ 387 der Gütezeichengemeinschaft. Aus den Prüfbestimmungen der RAL kann entnommen werden, wie der Druck eines Kompressionsstrumpfes auf ein Bein zu bestimmen ist. Als Messmittel, insbesondere Kompressionsprüfgerät, wird die Prüfung am HOSY Messgerät (Institut Hohenstein) vorgeschlagen. Die Prüfung erfolgt anhand der Messung der Spannkraft in den mehreren Messpunkten, welche abhängig von der jeweiligen Dehnbarkeit des Gestricks, also abhängig von der Elastizität des Gestricks, variiert. Aus der Spannkraft wird die Kompression errechnet. D.h. je mehr Elastizität ein Gestrick in Gestrickquerrichtung aufweist, desto geringer ist die durch das Gestrick am Patienten ausgeübte Kompression. Andererseits je geringer die Elastizität des Gestrickteils ist, desto höher ist die am Patienten ausgeübte Kompression.

Aus dem Stand der Technik sind eine Vielzahl von, insbesondere rundgestrickten, Gestrickteilen, insbesondere in Form von Arm- oder Beintrümpfen, bekannt.

Ein vorzugsweise kompressives Rundgestrick zum Überziehen über eine ein Gelenk aufweisende Extremität ist beispielsweise aus der EP 2 792 774 B1 bekannt.

Dieses bekannte kompressive Rundgestrick, welches es zur Aufgabe hat einen besseren Tragekomfort zu gewährleisten, umfasst einen Grundgestrickabschnitt, welcher eine Zone mit mehreren sich aneinander anschließenden Gestrickabschnitte umfasst. Insbesondere sind zwei in Gestricklängsrichtung voneinander beabstandete erste Gestrickabschnitte, die mit Flottungen gestrickt sind, vorgesehen, zwischen denen ein Gestrickbereich bestehend aus weiteren in Umfangsrichtung aneinander anschließenden unterschiedlichen Gestrickabschnitten angeordnet sind, welche unterschiedliche Flottungszahlen aufweisen und deren Flottungen einer Maschenreihe zur nächsten Maschenreihe versetzt gestrickt sind. Ferner ist ein Abschnitt vorgesehen der glatt gestrickt ist. Am oberen und unteren Ende weist das kompressive Rundgestrick einen Bund auf, welcher angenäht oder angestrickt ist.

Insgesamt wird somit ein Rundgestrick mit einem Gestrickabschnitt offenbart, der aus vier unterschiedlich gestrickten Gestrickbereichen besteht, wodurch ein anatomisch geformtes Gestrick realisiert wird, das einen erhöhten Tragekomfort aufweist.

Als nachteilig der aus diesem Stand der Technik bekannten Ausgestaltung des textilen Flächengebildes erweist sich, dass zwar durch das Einarbeiten der offenbarten Komfortzone das Gestrick bereichsweise eine Entlastungszone erhält, jedoch eine echte Anpassung des Grundgestrickteils an die anatomischen Erfordernisse nicht erfolgt. Das Grundgestrick wird nämlich durchgängig, insbesondere röhrenförmig, rundgestrickt, ohne der Anatomie einer Extremität tatsächlich angepasst zu sein. Die genannten Entlastungszonen ermöglichen nämlich lediglich eine leichtere Anpassung des gestrickten Strumpfes an die Extremität, was jedoch wiederum zu einer erhöhten Faltenbildung führt.

Ein weiterer Nachteil des aus dem Stand der Technik bekannten textilen Flächengebildes ist, dass die tatsächliche Lage des Gestricks am Körper und die tatsächliche Anatomie der Extremität nicht berücksichtig werden. Zudem wird das Grundgestrick, also dessen stricktechnischer Aufbau, insbesondere Bindungsart, in jenen Bereichen geändert, die eine erhöhte Anpassung des Gestrickteils an die Form der Extremität benötigen. Dies hat den Nachteil, dass sich die Gestrickeigenschaften ändern und möglicherweise auch verschlechtern, um die Passform zu erhöhen.

Schließlich erweist sich auch als nachteilig, dass die Anordnung des Bundes am Rundgestrick sowie die Lage des Bundes zu der Extremität eines Trägers gänzlich unberücksichtigt bleibt. Bundabschnitte weisen durch ihre einlagige oder doppellagige Ausbildung, vorzugsweise mit einer sich zum restlichen Grundgestrick unterscheidenden Grundbindungsart, insbesondere in Form von Rippen, einen erhöhten Platzbedarf auf, was sich wiederum als besonders störend beim Tragen des Gegenstandes auswirken kann, insbesondere auch dann, wenn dieser an der Extremität falsch platziert wird bzw. falsch zum Liegen kommt.

Aus der DE 10 2006 048 313 A1 ist ferner ein Kompressionsstrumpf aus einem elastischen Material, welches ein Gewebe, ein Gewirke oder ein Gestrick sein kann, bekannt. Am oberen und unteren Ende des Strumpfes kann ein Bündchen vorgesehen sein. Das obere Bündchen ist vorzugsweise gewinkelt an dem Kompressionsstrumpf angeordnet. Zur Ausbildung eines derartigen Bündchens, zumindest des unteren Bündchens, wird vorgeschlagen diesen als abgesaumten Bund auszugestalten. Bei einem solchen gesaumten Bund bzw. Saum wird die Gewebe- bzw. Gestrickkante umgeschlagen und an den übrigen Gewebe- bzw. Gestrickabschnitt angenäht.

Als nachteilig dieser aus diesem Stand der Technik bekannten Ausgestaltung der Kompressionsstrumpfhose, insbesondere eines derartigen Bündchens, erweisen sich die erhöhten Herstellkosten. Das Anbringen einer Naht zur Befestigung und Ausbildung des Saums stellt einen zusätzlichen und meist manuellen Prozessschritt dar, welcher Zusatzkosten verursacht.

Aus der DE 1 802 491 A1 ist eine gewirkte oder gestrickte Strumpfhose bekannt, welche aus einem Beinteil, einem Hosenteil und einem elastischen Abschlussbund besteht. Der Abschlussbund ist als Gummibund ausgebildet und an dem Hosenteil angebracht. Um die Passform der Strumpfhose der Anatomie des Trägers anzupassen, wird vorgeschlagen, die Strumpfhose am hinteren Teil höher auszubilden als am vorderen Teil. Hierzu wird ein keilförmiger Abschnitt in die Strumpfhose eingearbeitet, oder die Anzahl der Maschenreihen im hinteren Bereich des Hosenteils erhöht.

Als nachteilig dieser aus diesem Stand der Technik bekannten Ausgestaltung des Gestrickteils erweist sich der weiterhin schlechte Tragekomfort. Der Tragekomfort wird zwar durch die gewinkelte Anordnung des Bundes verbessert. Bei einem Gummibund handelt es sich jedoch üblicherweise um ein gewirktes, elastisches und meist einlagiges Gewebe, das an einen Gestrickabschnitt angenäht wird. Das dünne gewirkte Gewebe verursacht hierbei meist Einschnürungen beim Träger der Strumpfhose. Ein weiterer Nachteil ist, dass das Anbringen des Gummibunds an den Gestrickabschnitt ebenfalls einen zusätzliche Fertigungsschritt erfordert, nämlich das manuelle Annähen des Bundes, welcher zusätzliche Fertigungskosten mit sich bringt und somit die Strumpfhose verteuert.

Aus der DE1 760 82 2A1 ist zudem ein gewirktes Kleidungsstück bekannt. An einem unteren Ende bzw. Rand des Kleidungsstücks ist ein auf einer Wirkmaschine hergestellter und umgelegter Doppelrand vorgesehen. Dieser gebildete Saum wird dadurch erzeugt, dass dieser glatt gewirkt, umgelegt und anschließend angenäht wird.

Als nachteilig dieser aus diesem Stand der Technik bekannten Ausgestaltung des Kleidungsstücks mit einem schrägen Abschlussrand erweist sich auch hier, dass der untere Rand des Kleidungsstücks durch einen Saum ausgebildet ist. Die Herstellung des Saums erfordert, wie bereits oben erwähnt, das Einarbeiten einer Naht zur Ausbildung und Befestigung des Saums, was einen zusätzlichen Fertigungsschritt mit sich bringt. Hierdurch werden die Herstellkosten erhöht.

Der vorliegenden Erfindung liegt daher die Aufgabe zugrunde ein Gestrickteil, bevorzugt ein kompressives Gestrickteil, zu schaffen, welches die Nachteile aus dem Stand der Technik vermeidet, insbesondere die Passform eines Gestrickteils deutlich verbessert, dies bei einer gleichzeitig kostengünstigen und effizienten Herstellung des Gestrickteils.

Erfindungsgemäß besteht das Gestrickteil aus einem als Rundgestrick über mehrere Maschenreihen gestrickten Grundgestrickabschnitt sowie zumindest einen sich an den Grundgestrickabschnitt anschließenden, an diesen angestrickten und ebenfalls über mehreren Maschenreihen gestrickten Bundabschnitt, wobei die Maschenreihen des Grundgestrickabschnitts und des Bundabschnitts gewinkelt zueinander angeordnet sind. Der Bundabschnitt verläuft somit gewinkelt zu dem Grundgestrickabschnitt des Gestrickteils. Es änderst sich somit die Strickrichtung beider Abschnitte zueinander. Dies wird bevorzugt dadurch erzielt, dass eine oder mehrere zusätzliche sich in Umfangsrichtung weniger als 360 Grad erstreckende Teilmaschenreihen in bzw. an den Grundgestrickabschnitt ein- bzw. angestrickt werden. Der daran angestrickte Bundabschnitt ist dadurch in einem Winkel von 1 bis 90 Grad, bevorzugt in einem Winkel von 5 bis 45 Grad, an dem Grundgestrickabschnitt anordenbar.

Gemäß einem Ausführungsbeispiel kann der Grundgestrickabschnitt des Gestrickteils zur gewinkelten Anordnung des Bundabschnitts eine oder mehrere Grundstrickmaschenreihen aufweisen, deren Maschen in Umfangsrichtung zumindest teilweise eine geänderte Maschengröße aufweisen. Hierbei nimmt die Maschengröße vorzugsweise jeweils über mehrere Maschenreihen hinweg in Umfangsrichtung zu bzw. ab.

Gemäß einem weiteren Ausführungsbeispiel ist der Bundabschnitt des Gestrickteils doppellagig ausgebildet. Hierbei umfasst der Bundabschnitt eine gestrickte äußere Lage sowie eine zweite gestrickte innere Lage, wobei die beiden Lagen durchgängig gestrickt sind. Des Weiteren umfasst der Bundabschnitt bevorzugt eine sich vom Grundgestrickabschnitt unterscheidende Grundbindungsart auf. Bevorzugt ist der Bundabschnitt mit einer Rippenstruktur gestrickt. Um die Haftwirkung des Bundabschnitts zu erhöhen, weist dieser bevorzugt einen Haftabschnitt auf. Dieser kann durch eine auf das Grundgestrick aufgebrachte Beschichtung oder durch mehrere aufgebrachte Haftpartikel ausgebildet sein. Alternativ können auch Haftgarne in das Grundgestrick des Bundabschnitts eingestrickt sein, die an der Oberfläche des Grundgestricks angeordnet sind oder erhaben zum Liegen kommen. Zudem weist er gemäß diesem und der vorherigen Ausführungsbeispiele eine Breite von bevorzugt ungefähr 1 bis 5 cm auf. Besonders bevorzugt beträgt die Breite bei einem Beinbekleidungsstück ca. 2 cm und bei einem Armbekleidungsstück ca. 5 cm. Die Elastizität in Längs- und/oder Querrichtung des Bundabschnitts ist vorzugsweise geringer als jene des Grundgestrickabschnitts, so dass ein besonders guter Halt des Gestrickteils an der Extremität des Trägers gewährleistet wird.

Gemäß einem weiteren Ausführungsbeispiel weist das Gestrickteil, insbesondere der Grundgestrickabschnitt, an dessen gegenüberliegenden Ende, einen zweiten Bundabschnitt auf, welcher hinsichtlich seiner technischen Eigenschaften und Größe dem ersten Bundabschnitt entspricht oder sich von diesem unterscheidet.

Gemäß einem weiteren Ausführungsbeispiel des Gestrickteils schließt sich an den Grundgestrickabschnitt, insbesondere an dessen gegenüberliegenden Ende, eine geschlossene Zehenbox an, so dass der Grundgestrickabschnitt und die Zehenbox einen Strumpf mit geschlossener Spitze bilden. Weitere Funktionszonen, neben der Zehenbox, insbesondere eine Ferse, vorzugsweise eine Pendelferse, eine Entlastungszone, beispielsweise für den Knöchel eines Trägers, eine Übergangszone, oder eine Plüschzone können ebenfalls an dem Grundgestrickabschnitt vorgesehen sein. Bei Ausbildung einer Plüschzone ist diese bevorzugt im Bereich der Fußsohle des Strumpfes, der Zehenbox und/ oder der Ferse des Strumpfes angeordnet.

Gemäß einem weiteren Ausführungsbeispiel des Gestrickteils weist der Grundgestrickabschnitt zumindest einen Kompressionsbereich auf, wobei die Kompressionsstärke in dem genannten Bereich vorzugsweise zwischen 10 und 50 mmHg beträgt. Zudem nimmt die Kompressionsstärke in dem Kompressionsbereich, vorzugsweise kontinuierlich, in Gestrickslängsrichtung ab oder zu. Der Grundgestrickabschnitt weist somit bevorzugt einen graduellen Druckverlauf auf. Der graduelle Druckverlauf wird bevorzugt durch die Art und Weise, insbesondere über die Vorspannung eines Schussfadens beim Einbringen in das mit einem Grundstrickfaden gestrickte Gestrickteil und/ oder über die Anzahl von aufeinanderfolgenden Maschenreihen, in denen der Schussfaden hinterlegt wird, bestimmt.

Der vorzugsweise in Gestricklängsrichtung abfallende Druckverlauf des Grundgestrickabschnitts nimmt vorzugsweise in dem sich an den Grundgestrickabschnitt anschließenden Bundabschnitt zu. Dies erfolgt vorzugsweise sprungartig, d.h. über wenige Maschenreihen hinweg erhöht sich die Kompressionsstärke deutlich, also um vorzugsweise mehr als 3 mm Hg. Hierzu wird vorzugsweise der Schussfaden bezogen auf den Grundgestrickabschnitt mit einer erhöhten Vorspannung in das Gestrick des Bundabschnitts eingebracht oder es wird die Anzahl an Maschenreihen, in denen der Schussfaden eingelegt ist, in Gestricklängsrichtung erhöht. Letzteres kann auch dadurch erfolgen, dass der Schussfaden bei einem doppellagigen Bundabschnitt in beide Lagen, also der inneren und äußeren Lage, des Bundes eingebracht wird. Die erhöhte Kompressionsstärke im Bundabschnitt gewährleistet einen besseren Halt des Gestrickteils am Träger. Natürlich ist es auch denkbar, dass die Kompressionssträke im Bundabschnitt dem Verlauf bzw. Trend der Kompressionsstärke in dem benachbarten Grundgestrickabschnitt weiter folgt und dadurch konstant bleibt, weiter ansteigt, oder eben weiter abfällt.

Gemäß einem weiteren Ausführungsbeispiel des Gestrickteils, insbesondere bei Ausbildung des Gestrickteil als Beinbekleidungsstück ist der Bundabschnitt derart ausgebildet bzw. an dem Grundgestrickabschnitt angeordnet, dass dieser im getragenen Zustand im Wesentlichen dem Verlauf der Groß- und Kleinzehenballen eines Trägers folgt und/ oder diese überlappt.

Gemäß einem nicht erfindungsgemäßen Beispiel, insbesondere bei Ausbildung des Gestrickteils als Armbekleidungsstück, ist der Bundabschnitt derart ausgebildet, dass dieser im getragenen Zustand an der Armaußenseite sich weiter in Richtung der Schulter des Trägers erstreckt, als an der Arminnenseite. Bei Ausbildung des Gestrickteils als Unterarmstrumpf kommt somit der Bund im getragene Zustand an der Arminnenseite bevorzugt unterhalb der Ellenbeuge zum Liegen, wobei er sich an der Armaußenseite weiter in Richtung Schulter des Trägers erstreckt. Es wird durch die gewinkelte Anordnung des Bundes am Grundgestrickabschnitt ermöglicht, den Unterarm eines Trägers möglichst umfassend zu bedecken, aber gleichzeitig die Ellenbeuge frei zu halten, um einen besseren Tragekomfort zu gewährleisten. Bei Ausbildung des Armbekleidungsstücks als Oberarmstrumpf kann dies ebenso im Bereich der Achsel umgesetzt werden. So wird die Achsel frei gehalten, da der Bund vorzugsweise im getragenen Zustand an der Arminnenseite unterhalb der Achselhöhle zum liegen kommt, sich aber an der Armaußenseite weiter in Richtung Schulter erstreckt.

Das Gestrickteil nach den vorherigen Ausführungsbeispielen ist erfindungsgemäß Beinstrumpf, insbesondere als Socke, Beinling, Legging oder Hose, Bandage, oder als ein Strickteil einer Orthese ausgebildet. Besonders bevorzugt ist es als ein kompressives Gestrickteil ausgebildet, wobei die Kompressionsstärke in Gestricklängsrichtung, insbesondere von der Fessel in Richtung der Wadenmuskulatur, ab oder zunimmt.

Im Falle eines kompressiven Beinbekleidungsstücks mit einem Fußteil, betragen die durch das Gestrickteil erzeugten kompressiven Drücke in einem Fesselbereich des Beinbekleidungsstücks bevorzugt zwischen 10 und 50 mmHg, im Wadenbereich bevorzugt zwischen 5 und 30 mmHg und in einem Mittelfußbereich bevorzugt zwischen 10 und 30 mmHg.

Das vorliegende Beinbekleidungsstück zeichnet sich durch eine Reihe erheblicher Vorteile aus.

Durch die Ausbildung des Gestrickteils mit einem Grundgestrickabschnitt und einem Bundabschnitt, wobei die beiden Abschnitte gewinkelt zueinander angeordnet sind, wird die Passform des Gestrickteils wesentlich verbessert. Die teilweise Vorverlagerung des Bundes, insbesondere in medialer oder lateraler und/ oder proximaler oder distaler Richtung bezogen auf die Extremität eines Trägers, verleiht dem Gestrickteil eine der Anatomie der Extremität sehr stark angenäherte anatomische Form.

Es wird ferner durch das erfindungsgemäße Gestrickteil eine verbesserte Passform gewährleistet, ohne dass das Grundgestrickteil an die Form der Extremität besonders stark verdehnt werden muss. Eine Faltenbildung wird somit vermieden. Dies wird insbesondere dadurch erzielt, dass sich die Strickrichtung des Bundabschnitts bezogen auf die Strickrichtung des Grundgestrickabschnitts ändert. D.h., dass die Lage der Maschenreihen des Grundgestrickabschnitts und des Bundabschnitts gewinkelt zueinander angeordnet sind und somit auch gewinkelt an der Extremität eines Trägers zum Liegen kommen. Daraus folgt auch, dass die Verteilung der kompressiven Drücke des Gestrickteils besonders gleichmäßig ausfällt.

Besonders vorteilhaft ist insbesondere die Möglichkeit, bei der erfindungsgemäßen Ausgestaltung des Gestrickteils als Beinbekleidungsstück mit einer gewinkelten Anordnung des Bundabschnitts an dem Grundgestrickabschnitt, dass der Bundabschnitt im getragenen Zustand des Gestrickteils im Wesentlichen dem Verlauf der Groß- und Kleinzehenballen eines Trägers folgt bzw. diesen überlappt. Hierdurch und insbesondere durch die Ausbildung des Bundabschnitts als doppellagigen Bund ist es möglich, die Groß- und Kleinzehenballen zu polstern. Zudem kann durch eine zusätzliche Änderung der Strickrichtung und der daraus folgenden zusätzlichen Rückverlagerung des Bundes in proximaler Richtung, die Bewegungsfreit der Zehengelenke in proximaler Richtung beibehalten werden, was insbesondere bei kompressiven Beinbekleidungsstücken eine wichtige Rolle spielt.

Einen weiteren Vorteil der Erfindung bildet die kostengünstige Herstellung des einer Anatomie einer Extremität besonders gut angepassten Gestrickteils, welches einen gestrickten vorzugsweise doppellagigen Bundabschnitt, aufweist. Eine zusätzliche Konfektionierung des Bundes zur Verbesserung der Passform des Gestrickteils ist nicht notwendig. Das Gestrickteil kann in einem einzigen Fertigungs- und Strickprozess hergestellt werden, dies insbesondere dadurch, dass der Grundgestrickabschnitt und der einlagige oder doppellagige Bundabschnitt in einem einzigen Strickprozess durchgestrickt sind.

Nachfolgend wird die Erfindung anhand mehrerer Ausführungsbeispiele und in Verbindung mit den beigefügten Zeichnungen erläutert.

Dabei zeigt:
Figur 1 ein erstes Ausführungsbeispiel des erfindungsgemäßen Gestrickteils, ausgebildet als Beinbekleidungsstück, insbesondere als Beinstrumpf, in der Seitenansicht,
Figur 2 ein Beispiel eines nicht erfindungsgemäßen Gestrickteils, ausgebildet als Armbekleidungsstück, insbesondere als Armstrumpf, ebenfalls in der Seitenansicht,
Figur 3 ein weiteres Ausführungsbeispiel des erfindungsgemäßen Gestrickteils, erneut ausgebildet als Beinbekleidungsstück, insbesondere als Beinstrumpf mit offener Spitze, in der Vorderansicht,
Figur 4 ein Beispiel eines nicht erfindungsgemäßen Gestrickteils in Form eines Beinstrumpfes mit einem geschlossenen Zehenbereich in der Vorderansicht,
Figur 5 ein Maschenbild eines Ausschnitts A aus dem Beispiel des Gestrickteils gemäß Figur 2;

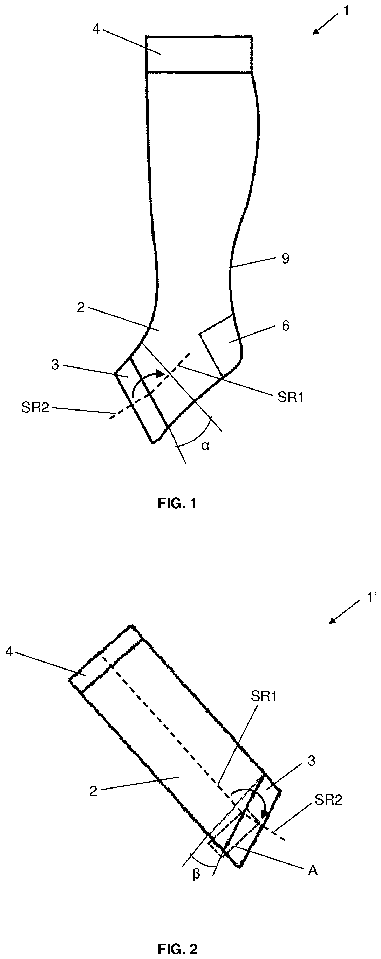

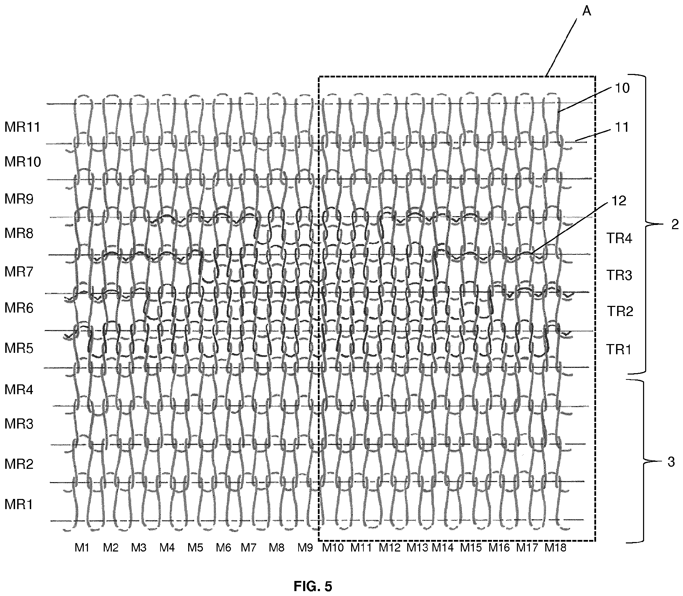

In Figur 1 ist ein erstes Ausführungsbeispiel des Gestrickteil 1 in Form eines Beinstrumpfes, insbesondere eines vorzugsweise kompressiven medizinischen Strumpfes oder Sportstrumpfes, in der Seitenansicht gezeigt. Der Beinstrumpf 1 besteht aus einem als Rundgestrick über mehrere Maschenreihen gestrickten Grundgestrickabschnitt 2 sowie zumindest einen sich an den Grundgestrickabschnitt 2 anschließenden und ebenfalls über mehreren Maschenreihen gestrickten Bundabschnitt 3. Am oberen Ende des Grundgestrickabschnitts 2, also dem Beinteil des Strumpfes, weist der Strumpf 1 bevorzugt einen zweiten Bundabschnitt 4 auf. Ferner ist in den Grundgestrickabschnitt 2 eine Ferse, vorzugsweise eine Pendelferse 6, eingestrickt. Gemäß einem bevorzugten Ausführungsbeispiel handelt es sich bei dem Strumpf 1 um einen kompressiven Strumpf 1, dessen erzeugte kompressiven Drücke in einem Fesselbereich 9 bevorzugt zwischen 10 und 50 mmHg, und in einem Wadenbereich zwischen 5 und 30 mmHg betragen. Die Kompressionsstärke nimmt hierbei vom unteren Ende in Richtung der Wadenmuskulatur bevorzugt ab. Der graduelle Druckverlauf wird bevorzugt durch die Art und Weise der Einbringung eines Schussfadens 12 in das mit einem Grundstrickfaden 10 gestrickte Gestrickteil 1 bestimmt, insbesondere über die Anzahl von aufeinanderfolgenden Maschenreihen, in den der Schussfaden 3 eingelegt ist, wie in Figur 5 gezeigt. Die kompressiven Werte werden mittels der eingangs vorgestellten Messmethodik und Messgerät, insbesondere mittels der Prüfung am HOSY Messgerät (Institut Hohenstein), gemessen.

Um nun einen sicheren Halt des Strumpfes 1 am Bein des Trägers zu gewährleisten, kann in das Gestrickteil 1, insbesondere in den Bundabschnitten 3 und/ oder 4, ein Haftfaden eingestrickt sein. Bei dem Haftfaden handelt es sich bevorzugt um einen Silikonfaden oder einen Faden, welcher ebenso eine haftende, bevorzugt leicht klebrige, adhäsive, jedenfalls rutschhemmende Oberfläche aufweist. Den Bundabschnitten 3 und/ oder 4 wird so an den Innen- und/ oder Außenseiten eine Haftschicht angebracht.

Der Bund 3 ist nun gemäß der Erfindung gewinkelt an dem Grundgestrickabschnitt 2 angeordnet bzw. gewinkelt mit diesem verstrickt. So verläuft der Bundabschnitt 3 in einem Winkel α von vorzugsweise größer als 5 Grad und kleiner als 90 Grad zu dem Grundgestrickabschnitt 2. Hierzu weist der Grundgestrickabschnitt 2 beispielsweise (1) eine oder mehrere zusätzliche sich in Umfangsrichtung weniger als 360 Grad erstreckende Teilmaschenreihen und/ oder (2) eine oder mehrere Grundstrickmaschenreihen auf, deren Maschen in Umfangsrichtung zumindest teilweise eine geänderte Maschengröße aufweisen. Hierdurch wird die Strickrichtung SR1 des Grundgestrickabschnitts 2 geändert, so dass der Bund 3 mit einer zweiten von der ersten Strickrichtung SR1 abweichenden Strickrichtung SR2 gestrickt wird. Das erfindungsgemäße Gestrickteil 1 weist dadurch einen Bundabschnitt 3 auf, der auf Grund seiner besonderen Anordnung zum Grundgestrickabschnitt 2 dem erfindungsgemäßen Gestrickteil 1 eine der Anatomie der Extremität angenäherte anatomische Form verleiht. Insbesondere ist durch die erfindungsgemäße Anordnung des Bundes 3 an dem Grundgestrickabschnitt 2 im Bereich der Fußsohle oder alternativ auch im Bereich von Gelenken, insbesondere an den Gelenkinnen- und der Gelenkaußenseiten, ein deutlich verbesserter Tragekomfort gegeben. Gemäß Figur 1 ist der Bund 3 des erfindungsgemäßen Gestrickteils 1 derart an dem Grundgestrickabschnitt 2 angeordnet, dass dieser an der Fußunterseite eines Trägers gegenüber der Fußoberseite vorgelagert ist. D.h. der Bund 3 ist in einem getragenen Zustand des Gestrickteils 1 weiter vorne in Richtung Fußspitze eines Trägers an der Fußunterseite angeordnet als an der Fußoberseite. Dies bringt den Vorteil mit sich, dass beispielsweise der Bund 3 die Groß- und Kleinzehenballen eines Fußes eines Trägers überlappt und diese polstert, ohne die Bewegungsfreit der Zehengelenke in proximaler Richtung zu beschränken, was insbesondere bei kompressiven Beinbekleidungsstücken eine wichtige Rolle spielt.

Besonders vorteilhaft ist es, wenn der Bundabschnitt 3 zusätzlich doppellagig ausgebildet bzw. doppellagig gestrickt ist, da er dadurch besonders hohe Polstereigenschaften aufweist. Die Breite des Bundes 3 beträgt vorzugsweise 1 bis 5 cm, besonderes bevorzugt 2 cm. Der Bund 3 zeichnet sich ferner bevorzugt dadurch aus, dass dieser eine geringere Elastizität in Längs- und/oder Querrichtung als der Grundgestrickabschnitt 2 aufweist.

Figur 2 zeigt ein nicht erfindungsgemäßes Beispiel des rundgestrickten Gestrickteils 1', nämlich ausgebildet als Armstrumpf 1'. Bei dem Armstrumpf 1' handelt es sich ebenfalls bevorzugt um ein kompressives Kleidungsstück, welches kompressive Drücke auf den Träger des Strumpfes 1', insbesondere auf dessen Ober und/ oder Unterarm ausübt. Hierzu weist der Armstrumpf 1' einen Grundgestrickabschnitt 2 sowie ebenfalls einen daran gewinkelt angeordneten Bundabschnitt 3 auf. Der Bundabschnitt 3 verläuft in einem Winkel β von vorzugsweise größer als 5 Grad zu dem Grundgestrickabschnitt 2. An dem gegenüberliegenden Ende ist ebenfalls, wie bereits in Figur 1 gezeigt, ein zweiter Bundabschnitt 4 vorgesehen.

Durch die gewinkelte Anordnung des Bundabschnittes 3, insbesondere durch die Änderung der Strickrichtung SR1 des Grundgestrickabschnitts 2, so dass der Bund 3 mit einer zweiten von der ersten Strickrichtung SR1 abweichenden Strickrichtung SR2 gestrickt wird, wird durch den Armstrumpf 1' ein besserer Tragekomfort gewährleistet, insbesondere wenn dieser als Unterarmstrumpf oder als Oberarmstrumpf ausgebildet ist. Durch die teilweise Vorverlagerung des Bundes 3 derart, dass dieser im getragenen Zustand an der Armaußenseite eines Trägers sich weiter in Richtung der Schulter des Trägers erstreckt, als an der Arminnenseite, wird gewährleistet, dass insbesondere auf Höhe der Ellenbeuge oder Achselhöhle, der Strumpf 1' diese Bereiche außenseitig überlappt, jedoch an der Arminnenseite nicht in diese hineinragt und die Bewegungsfreiheit dadurch einschränkt.

Der Abschnitt A kennzeichnet jenen Bereich, welcher in Figur 5 in Form eines Maschenbildes gezeigt wird.

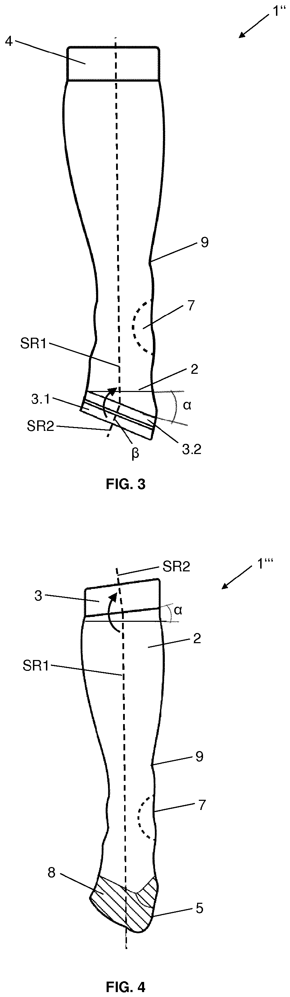

In Figur 3 ist ein weiteres nicht erfindungsgemäßes Beispiel des Gestrickteils 1" gezeigt, welches ebenfalls als Beinbekleidungsstück, insbesondere als Beinstrumpf 1" mit einer offenen Spitze und einem zweiten Bundabschnitt 4, ausgebildet ist. In Figur 3 wird ein weiteres besonders bevorzugtes Beispiel der gewinkelten Anordnung eines Bundes 3 gegenüber einem Grundgestrickabschnitt 2 gezeigt.

Der vorzugsweise ebenfalls kompressiv, wie in Figur 1 beschrieben, ausgebildete Beinstrumpf 1" umfasst neben dem Grundgestrickabschnitt 2 ebenfalls einen zweiten Bund 4 sowie zumindest einen Kompressionsbereich 9 im Bereich der Fessel eines Trägers, welcher sich bis zum oberen Strumpfende erstreckt. Ferner ist eine Funktionszone, insbesondere eine Entlastungszone 7, im Bereich des Knöchel eines Trägers vorgesehen. Die Entlastungszone 7 ist dadurch gekennzeichnet, dass der Grundgestrickabschnitt 2 in diesem Bereich eine höhere Elastizität in Längs- und/oder Querrichtung als der benachbarte Grundgestrickabschnitt 2 aufweist.

Der in diesem Ausführungsbeispiel gewinkelt zu dem Grundgestrickabschnitt 2 angeordnete Bundabschnitt 3 ist, wie der Zeichnung zu entnehmen ist, zweifach gewinkelt zu dem Grundgestrickabschnitt 2 angeordnet. D.h. dass der Grundgestrickabschnitt 2 und der Bundabschnitt 3 zum einen in einem Winkel β von vorzugsweise zwischen 5 und 45 Grad gesehen zur Längsachse des Grundgestrickabschnitts 2, insbesondere des Fußteils des Strumpfes 1", wie in dem Ausführungsbeispiel in Figur 2 gezeigt, also eine bezogen auf den Fuß eines Trägers seitliche, also mediale oder laterale Änderung der Strickrichtung SR1, und zum anderen, wie in dem Ausführungsbeispiel in Figur 1 gezeigt, in einem Winkel α von vorzugsweise ebenfalls zwischen 5 und 45 Grad, also eine Änderung der Strickrichtung SR1 bezogen auf den Träger in proximaler Richtung zueinander angeordnet sind. Letzteres ist in der Figur 3 besonders gut daran zu erkennen, dass der unter Abschnitt 3.1. des Bundabschnitts 3 gegenüber dem oberen Abschnitt 3.2 des Bundabschnitts 3 vorverlagert ist, sich also weiter nach vorne in Richtung der Zehen eines Trägers erstreckt.

Die Strickrichtung SR2 des Bundabschnitts 3 ändert sich somit bezogen auf die Strickrichtung SR1 des Grundgestrickabschnitts 2 bezogen auf den Träger des Strumpfes in lateraler Richtung in proximaler Richtung. D.h. auch, dass die Lage der Maschenreihen des Grundgestrickabschnitts 2 und des Bundabschnitts 3 gewinkelt, vorzugsweise gemäß dem Ausführungsbeispiel 3, zueinander angeordnet sind. Das erfindungsgemäße Gestrickteil 1" weist dadurch einen Bundabschnitt 3 auf, der auf Grund seiner besonderen Anordnung zum Grundgestrickabschnitt 2 dem erfindungsgemäßen Gestrickteil 1 eine der Anatomie der Extremität sehr stark angenäherte anatomische Form verleiht. Besonders bevorzugt folgt bzw. überlappt der Bundabschnitt 3 hierbei im getragenen Zustand im Wesentlichen dem Verlauf der Groß- und Kleinzehenballen eines Trägers. Auch gemäß diesem Ausführungsbeispiel ist der Bundabschnitt 3 bevorzugt doppellagig ausgebildet ist, da er dadurch besonders hohe Polstereigenschaften aufweist.

In Figur 4 ist ein weiteres nicht erfindungsgemäßes Beispiel des Gestrickteils 1‴ abgebildet, welches ebenfalls als vorzugsweise kompressiver Beinstrumpf 1‴ ausgebildet ist, jedoch nun gemäß diesem Beispiel einen geschlossenen Zehenbereich aufweist. Der Beinstrumpf 1‴ weist somit einen Grundgestrickabschnitt 2 sowie eine distal daran angeordnete Zehenbox 5 auf. Am oberen, also proximalen, Ende ist ein Bundabschnitt 3 vorgesehen, welcher gemäß der Erfindung gewinkelt zu dem Grundgestrickabschnitt, also dem Bein- und/ oder Fußteil angeordnet ist. Neben einem Kompressionsbereich 9 und einer Entlastungszone 7, wie in den Figuren zuvor beschrieben, weist der Strumpf 1‴ bevorzugt zudem eine weitere Funktionszone, nämlich eine Polsterung 8 im Bereich der Zehenbox 5, also auch bevorzugt im Sohlenbereich des Strumpfes 1‴ auf. Diese ist bevorzugt als Plüsch, insbesondere als Sandwich Plüsch, ausgebildet. Hierzu ist im Zehen- und Sohlenbereich in das Grundgestrick bevorzugt zumindest ein weiterer Plüschhenkel ausbildenden Plüschfaden eingearbeitet. Dieser besteht vorzugsweise aus Polyamid, Polyester, Polypropylen oder Naturfaser. Hierdurch weist das Gestrickteil 1" eine polsternde Eigenschaft auf. Besonders bevorzugt erstreckt sich die zumindest eine Polsterung 8 neben der Fußsohle, also dem Zehenbereich, der Fußballen, sowie der Ferse im getragenen Zustand des Strumpfes 1‴ zusätzlich zumindest teilweise entlang der Achillessehne in Richtung Wade.

Gemäß diesem Beispiel und wie aus der Figur 4 zu entnehmen ist, verläuft der am oberen Ende des Strumpfes angeordnete Bundabschnitt 3 in einem Winkel β von vorzugsweise größer als 5 Grad zu dem Grundgestrickabschnitt 2. Auch hier wird durch die gewinkelte Anordnung des Bundabschnittes 3, insbesondere durch die Änderung der Strickrichtung SR1 des Grundgestrickabschnitts 2, so dass der Bund 3 mit einer zweiten von der ersten Strickrichtung SR1 abweichenden Strickrichtung SR2 gestrickt wird, ein besserer Tragekomfort gewährleistet. Durch die teilweise Vorverlagerung des Bundes 3 an der bezogen auf einen Träger medialen Seite des Beinstrumpfes 1‴, wird die Passform des Strumpfes 1‴ deutlich verbessert. Alternativ kann auch eine laterale Vorverlagerung des Bundes 3 von Vorteil sein.

Alternativ zu den zuvor gezeigten Ausführungsbeispielen ist es natürlich auch möglich, dass das erfindungsgemäße Gestrickteil als Socke, Bandage, oder als ein Strickteil einer Orthese ausgebildet ist.

Schließlich zeigt Figur 5 ein Maschenbild, insbesondere einen Ausschnitt A aus dem nicht erfindungsgemäßen Beispiel des Gestrickteils 1' gemäß Figur 2. Das Maschenbild zeigt den gestrickten Grundgestrickabschnitt 2 und Bundabschnitt 3, welche jeweils aus einem Grundstrickfaden 10, der maschenbildend über mehrere Strickreihen MR1 bis MR7 und Maschenstäbchen M1 bis M18 hinweg verstrickt ist, gebildet sind. Bevorzugt besteht der Grundstrickfaden aus Kunststoff, insbesondere Polyamid, Polyester, Polypropylen oder Polyurethan. Alternativ ist es möglich, dass dieser aus Naturfasern, insbesondere Wolle oder Zellulose bzw. Viskose, besteht. Die Maschenreihen MR1 bis MR4 zeigen einen Ausschnitt aus dem Bundabschnitt 3. Die Maschenreihen MR5 bis MR 11 zeigen einen Ausschnitt aus dem Grundgestrickabschnitt 2. In die Grundstrickmaschen ist in beiden Bereichen 2 und 3 zumindest ein kompressionsgebender Schussfaden 11 eingelegt. Der Schussfaden ist bevorzugt aus einem Elastomer, vorzugsweise auf PU-Basis. Der hochelastische Schussfaden 3 ist in diesem Ausführungsbeispiel in jeder Maschenreihe eingelegt. Dies ist jedoch beliebig ausbildbar. Er kann auch nur in jeder zweiten Maschenreihe oder in jeder n-ten Maschenreihe eingelegt sein. Des Weiteren kann der Schussfaden 11 in Umfangsrichtung gesehen auch abschnittsweise in einem oder mehreren Maschenstäbchen maschenbildend verstrickt sein.

Neben dem Grundstrickfaden 10 und dem Schussfaden 11 zeigt das Maschenbild, dass in den Grundgestrickabschnitt 2 zur gewinkelten Anordnung des Bundabschnitts 3, ein maschenbildender und somit Zusatzmaschenreihen TR1 bis TR4 bildender Zusatzfaden 12 eingestrickt ist. Dieser Faden 12 ist, insbesondere zur Änderung der Strickrichtung, nur abschnittsweise, also partiell und nicht komplett umlaufend, in Umfangsrichtung gesehen, in den Grundgestrickabschnitt 2 eingestrickt und an dessen Enden auch in den Grundstrickmaschenreihen, vorzugsweise mittels Fang und Flottung, verriegelt. Wie dem Maschenbild zu entnehmen ist, erstrecken sich die Zusatzmaschenreihen TR1 bis TR4 aus den Ausschnitt A, welcher die Oberseite des in Figur 2 gezeigten Gestrickteils 1' kennzeichnet, in den gezeigten benachbarten Ausschnitt und somit in die Unterseite des Gestrickteils 1' gemäß Figur 2. Ferner sind gemäß diesem Ausführungsbeispiel die Zusatzmaschenreihen TR1 bis TR4 unterschiedlich lang. Die Länge der Teilreihen nimmt mit zunehmendem Abstand zum Bundabsschnitt 2 ab. Dies hat sich zur Ausbildung der gewinkelten Anordnung beider Abschnitte 2 und 3 zueinander, als besonders vorteilhaft herausgestellt. Denkbar sind jedoch auch andere Ausführungen bezüglich der Zusatzmaschenreihenlänge. Durch die lokale und partielle Erhöhung der Maschenanzahl wird erfindungsgemäß die Strickrichtung geändert, wodurch es möglich ist, dass der Grundgestrickabschnitt 2 und der Bundabschnitt 3 gewinkelt zueinander angeordnet sind. Alternativ ist es auch denkbar, dass zur gewinkelten Anordnung des Bundabschnitts 3 eine oder mehrere Grundstrickmaschenreihen vorgehsehen sind, deren Maschen in Umfangsrichtung zumindest teilweise eine geänderte Maschengröße aufweisen.

## Patentansprüche

1. Gestrickteil (1', 1") bestehend aus einem als Rundgestrick über mehrere Maschenreihen gestrickten Grundgestrickabschnitt (2) sowie einen sich an den Grundgestrickabschnitt (2) anschließenden, an diesen angestrickten und ebenfalls über mehreren Maschenreihen gestrickten ersten Bundabschnitt (3), wobei das Rundgestrick ein Beinstrumpf (1, 1', 1") ist, der Grundgestrickabschnitt (2) eine Funktionszone, die als Ferse (6) ausgebildet ist, aufweist und sich an dem Grundgestrickabschnitt (2), an dessen gegenüberliegenden Ende, ein zweiter Bundabschnitt (4) anschließt, **dadurch gekennzeichnet, dass** der erste Bundabschnitt (3) im getragenen Zustand im Bereich der Fußsohle angeordnet ist und der Grundgestrickabschnitt (2) und der erste Bundabschnitt (3) derart gewinkelt zueinander angeordnet sind, dass dieser an der Fußunterseite eines Trägers gegenüber der Fußoberseite vorgelagert ist.

2. Gestrickteil (1', 1") nach Anspruch 1, **dadurch gekennzeichnet, dass** der Grundgestrickabschnitt (2) zur gewinkelten Anordnung des ersten Bundabschnitts (3) eine oder mehrere zusätzliche sich in Umfangsrichtung weniger als 360 Grad erstreckende Teilmaschenreihen aufweist.

3. Gestrickteil (1', 1") nach Anspruch 1, **dadurch gekennzeichnet, dass** der Grundgestrickabschnitt (2) zur gewinkelten Anordnung des ersten Bundabschnitts (3) eine oder mehrere Grundstrickmaschenreihen aufweist, deren Maschen in Umfangsrichtung zumindest teilweise eine geänderte Maschengröße aufweisen.

4. Gestrickteil (1', 1") nach einem der vorherigen Ansprüche, **dadurch gekennzeichnet, dass** der Grundgestrickabschnitt (2) und der erste Bundabschnitt (3) in einem Winkel (α, β) von 1 bis 90 Grad, bevorzugt in einem Winkel (α, β) von 5 bis 45 Grad, zueinander angeordnet sind.

5. Gestrickteil (1', 1") nach einem der vorherigen Ansprüche, **dadurch gekennzeichnet, dass** der erste Bundabschnitt (3) doppellagig ausgebildet ist.

6. Gestrickteil (1', 1") nach einem der vorherigen Ansprüche, **dadurch gekennzeichnet, dass** der erste Bundabschnitt (3) eine Breite von ungefähr 1 bis 5 cm aufweist.

7. Gestrickteil (1', 1") nach einem der vorherigen Ansprüche, **dadurch gekennzeichnet, dass** der erste Bundabschnitt (3) eine geringere Elastizität in Längs- und/oder Querrichtung als der Grundgestrickabschnitt (2) aufweist.

8. Gestrickteil (1', 1") nach einem der vorherigen Ansprüche, **dadurch gekennzeichnet, dass** der Grundgestrickabschnitt (2) zumindest einen Kompressionsbereich (9) aufweist, wobei die Kompressionsstärke in dem Bereich (9) zwischen 10 und 50 mmHg beträgt.

9. Gestrickteil (1") nach einem der vorherigen Ansprüche, **dadurch gekennzeichnet, dass** der erste Bundabschnitt (3) derart ausgebildet ist, dass dieser im getragenen Zustand im Wesentlichen dem Verlauf der Groß- und Kleinzehenballen eines Trägers folgen und/ oder diese überlappen kann.

## Claims

1. A knitted part (1', 1") consisting of a basic knitted section (2) knitted as a circular knit over several rows of stitches and at least one first band section (3) adjoining the basic knitted section (2), knitted onto this and also knitted over several rows of stitches, wherein the circular knit is a leg stocking (1, 1', 1"), the basic knitted section (2) comprises a functional zone which is configured as a heel (6) and a second band section (4) adjoins the basic knitted section (2) at its opposite end, **characterized in that** the first band section (3) in the worn state is arranged in the region of the sole of the foot and the basic knitted section (2) and the first band section (3) are arranged at an angle with respect to one another in such a manner that this is positioned on the underside of the foot of a wearer at the front with respect to the upper side of the foot.

2. The knitted part (1', 1") according to Claim 1, **characterized in that** the basic knitted section (2) has one or more additional partial stitch rows extending less than 360 degrees in the circumferential direction for the angled arrangement of the first band section (3).

3. The knitted part (1', 1") according to Claim 1, **characterized in that** for the angled arrangement of the first band section (3) the basic knitted section (2) has one or more basic knitted stitch rows, the stitches of which have at least partially a changed stitch size in the circumferential direction.

4. The knitted part (1', 1") according to one of the preceding claims, **characterized in that** the basic knitted section (2) and the first band section (3) are arranged at an angle (α, β) of 1 to 90 degrees, preferably at an angle (α, β) of 5 to 45 degrees, with respect to one another.

5. The knitted part (1', 1") according to one of the preceding claims, **characterized in that** the first band section (3) is configured to be double-layered.

6. The knitted part (1', 1") according to one of the preceding claims, **characterized in that** the first band section (3) has a width of preferably approximately 1 to 5 cm.

7. The knitted part (1', 1") according to one of the preceding claims, **characterized in that** the first band section (3) has a lower elasticity in the longitudinal and/or transverse direction than the basic knitted section (2).

8. The knitted part (1', 1") according to one of the preceding claims, **characterized in that** the basic knitted section (2) has at least one compression region (9), wherein the compression strength in the region (9) is between 10 and 50 mmHg.

9. The knitted part (1") according to one of the preceding claims, **characterized in that** the first band section (3) is configured such that, when worn, it essentially follows the course of the balls of the big and little toes of a wearer and/or overlaps them.

## Revendications

1. Pièce tricotée (1', 1"), constituée d'une partie (2) en tricot de base, tricotée en tricot tubulaire sur plusieurs rangées de mailles, ainsi que d'une première partie formant bande (3), se raccordant sur la partie (2) en tricot de base, tricotée à la suite de celle-ci et également tricotée sur plusieurs rangs de mailles, le tricot tubulaire étant une chaussette (1, 1', 1"), la partie (2) en tricot de base comportant une zone fonctionnelle, qui est conçue sous la forme d'un talon (6) et une deuxième partie formant bande (4) se raccordant sur la partie (2) en tricot de base, sur l'extrémité opposée de celle-ci, **caractérisée en ce qu'**à l'état porté, la première partie formant bande (3) est placée dans la zone de la plante du pied et la partie (2) en tricot de base et la première partie formant bande (3) sont placées sous forme coudée l'une par rapport à l'autre, de telle sorte que celle-ci soit placée en amont sur la face inférieure du pied d'un porteur, au regard de la partie supérieure du pied.

2. Pièce tricotée (1', 1") selon la revendication 1, **caractérisée en ce que** pour le placement coudé de la première partie formant bande (3), la partie (2) en tricot de base comporte un ou plusieurs rangs partiels de mailles supplémentaires, s'étendant sur moins de 360 degrés dans la direction circonférentielle.

3. Pièce tricotée (1', 1") selon la revendication 1, **caractérisée en ce que** pour le placement coudé de la première partie formant bande (3), la partie (2) en tricot de base comporte un ou plusieurs rangs de mailles de tricot de base, dont les mailles présentent au moins en partie dans la direction circonférentielle une dimension des mailles modifiée.

4. Pièce tricotée (1', 1") selon l'une quelconque des revendications précédentes, **caractérisée en ce que** la partie (2) en tricot de base et la première partie formant bande (3) sont placées l'une par rapport à l'autre sous un angle (α, β) de 1 à 90 degrés, de préférence sous un angle (α, β) de 5 à 45 degrés.

5. Pièce tricotée (1', 1") selon l'une quelconque des revendications précédentes, **caractérisée en ce que** la première partie formant bande (3) est conçue en double couche.

6. Pièce tricotée (1', 1") selon l'une quelconque des revendications précédentes, **caractérisée en ce que** la première partie formant bande (3) présente une largeur d'environ 1 à 5 cm.

7. Pièce tricotée (1', 1") selon l'une quelconque des revendications précédentes, **caractérisée en ce que** la première partie formant bande (3) fait preuve d'une élasticité moindre dans la direction longitudinale et / ou transversale que celle de la partie (2) en tricot de base.

8. Pièce tricotée (1', 1") selon l'une quelconque des revendications précédentes, **caractérisée en ce que** la partie (2) en tricot de base comporte au moins une zone de compression (9), la résistance à la compression étant de l'ordre (9) compris entre 10 et 50 mmHg.

9. Pièce tricotée (1") selon l'une quelconque des revendications précédentes, **caractérisée en ce que** la première partie formant bande (3) est conçue de telle sorte qu'à l'état porté, celle-ci puisse suivre sensiblement le trajet du coussinet du gros orteil et du petit orteil et / ou le chevaucher.
